(19) Europäisches Patentamt
European Patent Office
Office européen des brevets

(11) Publication number: **0 055 091**
**B1**

(12) # EUROPEAN PATENT SPECIFICATION

(45) Date of publication of patent specification: **31.07.85**

(21) Application number: **81305945.8**

(22) Date of filing: **18.12.81**

(51) Int. Cl.[4]: **C 08 G 12/30,** C 07 D 251/30, C 08 L 61/26, C 08 K 5/34

(54) **Hemiacetals of adducts of acrolein and isocyanuric acid and polymer compositions thereof.**

(30) Priority: **22.12.80 US 219018**
**22.12.80 US 219391**

(43) Date of publication of application:
**30.06.82 Bulletin 82/26**

(45) Publication of the grant of the patent:
**31.07.85 Bulletin 85/31**

(84) Designated Contracting States:
**BE DE FR GB IT**

(56) References cited:
**DE-C- 918 780**
**FR-A-1 339 408**
**GB-A- 542 974**
**US-A-3 737 432**

(73) Proprietor: **MONSANTO COMPANY**
**Patent Department 800 North Lindbergh Boulevard**
**St. Louis, Missouri 63166 (US)**

(72) Inventor: **Cohen, Saul Mark**
**15 Lindsay Road**
**Springfield Massachusetts 01128 (US)**
Inventor: **LeBlanc, John Roger**
**34 Decorie Drive**
**Wilbraham Massachusetts 01095 (US)**

(74) Representative: **Pearson, John Lionel et al**
**Monsanto Europe S.A. Avenue de Tervuren**
**270-272 Letter Box No 1**
**B-1150 Brussels (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to hemiacetals prepared by reaction of monohydric alcohols and adducts of acrolein and isocyanuric acid and to a process of preparation of such hemiacetals. In particular, this invention relates to hemiacetal compositions comprising hemiacetals of (3-oxopropyl)isocyanurates and to a process of preparation of such hemiacetals. It also relates to polymer compositions comprising polyfunctional hemiacetal-reactive compounds and the above-described hemiacetals as well as to the polymeric reaction products of such compositions and to articles comprising such reaction products.

The process of the invention for the preparation of a hemiacetal comprises:

a. slurrying isocyanuric acid in a solvent medium having a pH in the range of about 5 to about 7, the said medium containing at least about 1 mole of acrolein per mole of isocyanuric acid.

b. allowing the acrolein to add to the isocyanuric acid at a temperature in the range of about 50 to about 120°C to form a substantially clear solution; and

c. allowing the adduct to react with a monohydric primary or secondary alcohol at a temperature in the range of about 50 to about 80°C at a pH in the range of about 5 to about 7.

The hemiacetals contain various degrees of (3-hydrocarbyloxy-3-hydroxypropyl) substitution at the isocyanurate nitrogen atoms depending on the initial 3-oxypropyl content of the aldehyde adduct and the type and concentration of monohydric alcohol used in the reaction with the adduct. Preferably the adduct is formed from one mole of isocyanuric acid and from two to three moles of acrolein. The hemiacetals form stable solutions of low viscosity.

The solvent medium in step a. of the process of the invention contains at least about 1 mole of acrolein per mole of isocyanuric acid, and preferably in step b. at least about one mole of acrolein reacts with one mole of isocyanuric acid to form an aldehyde containing an average of about one 3-oxopropyl group per isocyanurate ring. More preferably at least about 2 moles of acrolein react with one mole of isocyanuric acid to form an aldehyde containing an average of about two 3-oxopropyl groups per isocyanurate ring, and most preferably about three moles of acrolein react per mole of isocyanuric acid to provide an aldehyde, containing tris(3-oxopropyl)isocyanurate as the major fraction, (hereinafter referred to as isocyanuric trialdehyde).

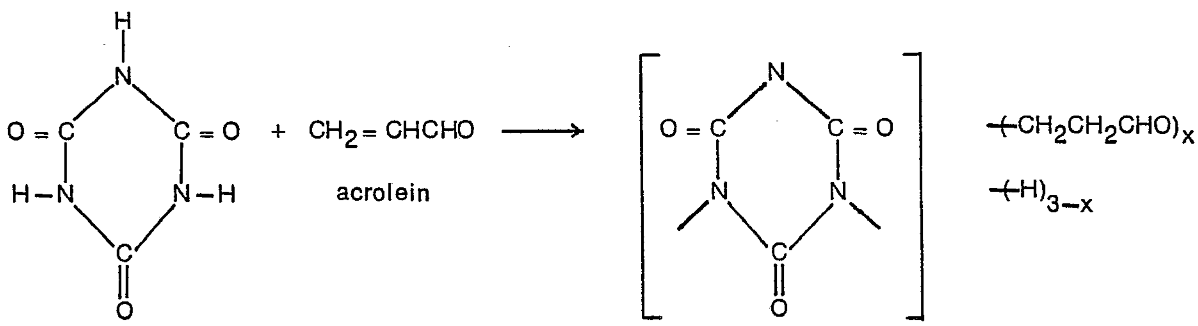


isocyanuric acid (3-oxopropyl)isocyanurates (x ≤ 3)

Under normal conditions, isocyanuric acid is the predominant tautomer in "cyanuric acid", comprising about 94 mole percent of the tautomeric mixture. In the reaction with acrolein, the iso-cyanuric acid tautomer reacts more readily than the cyanuric acid tautomer so that the reaction product may contain more than 94 percent of the isocyanurate derivative. However, to the extent that cyanurates are formed, since they contain an equal number of aldehyde groups, they contribute equally to the aldehyde functionality of the product aldehyde. For the purposes of this disclosure, it is understood that the term "isocyanuric acid" includes isocyanuric acid containing a minor amount of cyanuric acid tautomer.

Several commercial grades of "cyanuric acid" are available, containing various amounts of impurities such a ammelide and ammeline and ammelide- and ammeline-sulfamic acids. While any of these grades are suitable for the addition of acrolein, it is preferred that the "cyanuric acid" contain at least about 98 weight percent of the isocyanuric acid and cyanuric acid tautomers, and ammelide- and ammeline-sulfamic acids in the range of about 0.1 to about 0.3 weight percent.

The reaction temperature in step b. of the process is preferably in the range of about 80 to about 100°C, and more preferably in the range from about 88 to about 92°C to achieve a fast rate of reaction without excessive formation of insoluble polymer.

The solvent medium in the process can be acrolein in substantial excess over the stoichiometric amount for reaction with isocyanuric acid, or it can be selected from solvents for the aldehyde reaction product which are inert to aldehydes under the conditions of the reaction, and can optionally contain an excess of acrolein over the amount desired to be added to the isocyanuric acid. Excess acrolein can be used, especially when a high degree of acrolein addition and a fast reaction rate are desired. Thus, depending upon the desired degree of acrolein addition, the amount of acrolein can range from about 1

mole to about 6 moles or more per mole of isocyanuric acid. In general the range of solvents for the isocyanuric monoaldehyde is rather limited and includes solvents such as water, dimethylformamide, dimethylacetamide and methyl sulfoxide which are inert but highly polar. In contrast the range of solvents for the isocyanuric trialdehyde is quite broad and includes ethers, esters, ketones and hydrocarbons or mixtures thereof. Among the preferred solvents for the trialdehyde are the lower boiling ethers, esters and ketones since they can be readily evaporated when the reaction is complete.

Steps a. and c. of the process involve a pH in the range of about 5 to about 7. pH is measured by applying a few drops of reaction solution to universal pH paper or some comparable pH color indicator, allowing the solvent to evaporate and adding a few drops of methanol to the pH paper. When the methanol has evaporated, the color of the pH paper is compared with the color standard and the pH value thus obtained is considered to he the pH of the reaction solution.

Preferably the pH is maintained in the range of about 5 to about 7 by addition of a salt of an amine of $pK_b$ in the range of about 3.5 to about 9.5 and an acid of $pK_a$ in the range of about 1 to about 5.

As will be evident from well-established principles, the quantity of acid required for control of the acidity within the specified limits will be at least that required to react with all the amine plus an amount in excess thereof sufficient to keep the apparent pH of the reaction mixture below 7.0. As will also be evident from well-established principles, the amount of acid to be employed over and above that required for reaction with the amine will vary from acid to acid and amine to amine, dependent upon the dissociation constants of the compounds involved. Thus, in the case of a very weak amine and a strong acid, a slight amount of the acid over the stoichiometric equivalent would be sufficient. Conversely, with a strong amine base and weak acid, stoichiometric equivalents of acid to amine as high as 3 to 1 may be indicated. In the case of the amphoteric amine compounds, the use of acid can be dispensed with, but it is not necessary to do so provided the quantity used is insufficient to take the pH of the reaction outside the specified range. In some instances the use of a small quantity of acid with the amphoteric amine compound also may be found advantageous.

The amine can be selected from primary, secondary and tertiary amines, polyamines, and the like, including amphoteric amine compounds. These amines can be aliphatic or aromatic or mixed aliphatic and aromatic, including carbocyclic and heterocyclic. By the term "amphoteric amine compound", as used herein, is meant an amine of the classes listed above which also contains in the molecule an acidic hydrogen atom connected to a carbon atom through an oxygen atom, as in the case of the aminoacids and aminophenols by way of illustration. Representative of the useful amines are the following: monomethylamine, dimethylamine, trimethylamine, monoethylamine, diethylamine, triethylamine, monopropylamine, dipropylamine, tripropylamine monobutylamine, dibutylamine, tributylamine, and mono-, di-, and tri-alkyl amines containing up to seven carbon atoms to the alkyl group; aniline, diphenylamine, toluidine, monomethylaniline, dimethylaniline and other arylamines and alkyl-substituted arylamines containing up to seven carbon atoms to the group attached to the nitrogen atom; monoethanolamine, diethanolamine, triethanolamine, diethylene triamine, triethylene tetramine, phenylenediamines, and other aliphatic and aromatic polyamines containing not more than seven carbon atoms to the aliphatic or aromatic group present therein. Representative of the amphoteric amine compounds that can be used as catalyst in our process are glycine, beta-alanine, anthranilic acid and aminophenols.

The acid can be selected from a wide variety of acids including formic, acetic, propionic, butyric, valeric, hexanoic, heptanoic, octanoic, oxalic, succinic, glycolic, lactic, benzoic, phthalic and sulfamic.

In general, the preferred amines are tertiary amines such as trimethylamine, triethylamine and tripropylamine and the preferred acids are the lower carboxylic acids such as formic acid, glycolic acid and lactic acid.

The amine salt concentration is not narrowly critical but may be varied within moderate limits. A concentration in the range of about 0.1 to about 1.0 mole per hundred moles of isocyanuric acid is preferred. Within this range the exact amount employed depends to some extent on the particular amine salt selected and the reaction temperature. At concentrations below about 0.1 mole of amine salt per 100 moles of isocyanuric acid, an undesirable formation of acrolein oligomers competes with the formation of the desired adduct of isocyanuric acid, and crosslinked product also forms.

When the reaction has been carried to completion, the amine salt can be removed by treatment of the reaction solution with a suitable adsorbent or absorbent for the amine salt such as a diatomaceous earth, finely divided silica, alumina, animal charcoal, an ion-exchange resin or a combination of ion-exchange resins. The treatment can be carried out in any convenient way, such as by stirring the solution with the sorbent for sufficient time to substantially sorb the amine salt and filtering off the sorbent, or by passing the reaction solution through a bed of sorbent. After removal of the amine salt, sufficient acid of moderate strength with pKa in the range of about 1 to about 3 may be added to produce a final product with a pH in the range of about 1 to about 3 and more preferably in the range of about 1.5 to 2. Suitable acids include oxalic acid, dichloroacetic acid, glycerophosphoric acid, maleic acid, 2-methyl-6-nitrobenzoic acid and phosphorous acid. The solution of the aldehyde reaction product is then stripped to remove solvent. When the reaction solvent is provided by an excess of acrolein, removal of the excess acrolein is preferably achieved by a multicycle addition of inert solvent and removal of the solvent by vacuum stripping and finally water is added and removed by vacuum stripping. Suitable solvents for the stripping process are ethers, esters and ketones of boiling point in the range of about 60 to about 100°C.

Any commercial grade of acrolein is suitable for the reaction with isocyanuric acid. Such grades generally contain up to about 4 weight percent water. Also from about 0.1 to about 0.25 weight percent hydroquinone can be present as a polymerization inhibitor. While water up to about 4 percent is tolerable and has little effect on the acroleinisocyanuric acid reaction, we have found that when the water content is reduced to less than about 1.0 percent, the amount of polyacrolein produced during the reaction is decreased. The tendency of acrolein to dimerize by a Diels-Alder addition has long been recognized. Acrolein containing from about 0.1 to about 2.0 percent of dimer yields greater than the theoretical amount of the desired aldehyde upon reaction of the acrolein with isocyanuric acid. However, analysis by thin layer chromatography reveals no difference in the aldehyde prepared from such acrolein compared with aldehyde prepared by reaction of isocyanuric acid and acrolein containing less than 0.1 percent dimer.

In step c. of the process of this invention, the aldehyde adduct is converted to a monomeric hemiacetal by reaction with a monohydric primary or secondary alcohol. The reaction can be carried out merely by contacting the aldehyde adduct with the alcohol if the latter is liquid, or by stirring a mixture of the aldehyde and the alcohol in an inert solvent until a clear solution is obtained. Such inert solvents include ethers, esters and ketones. Preferably the solvent is also a solvent for the hemiacetal product. Since hemiacetal formation is an equilibrium reaction, it can be advantageous to use an excess of the alcohol in forming the hemiacetal. However, when a solid aldehyde is reacted with less than the amount of alcohol needed to cause substantially complete hemiacetalization, a degree of hemiacetalization depending upon the particular monohydric alcohol and its concentration takes place to provide a solution of the partially hemiacetalized isocyanuric aldehyde, stable at room temperature.

A pH in the range of about 5 to 7 can be obtained by adding an amine salt to the reaction medium as described hereinabove for the acrolein-isocyanuric acid reaction medium. The amine salt can be any of those listed hereinabove. The preferred amine salts are prepared from tertiary amines such as trimethylamine, triethylamine and tripropylamine and the lower fatty acids such as formic, acetic and propionic acids.

Within the range about 50 to about 80°C, the reaction temperature is not critical, and can be selected to provide a reasonable rate of solution of the aldehyde. The reaction temperature is preferably slightly above the glass transition temperature of the aldehyde. For example with isocyanuric trialdehyde, the temperature is preferably in the range of about 60—70°C, to obtain rapid solution and avoid possible crosslinking or other undesirable side reactions. When the reaction is complete the amine salt may be removed in the fashion described hereinabove for the preparation of the aldehydes.

While any monohydric primary or secondary alcohol or mixture of alcohols may be used in hemiacetalization, the $C_1$ to $C_8$ alcohols are preferably selected for the preparation of hemiacetals which are to be used as polymerization or crosslinking agents, for example methyl, ethyl, propyl, isopropyl, butyl, cyclohexyl and benzyl alcohols, and 2-alkoxyethanols such as 2-methoxyethanol and 2-butoxyethanol are preferred. Methyl alcohol is especially preferred.

When hemiacetalization of the aldehydes is carried out with excess monohydric alcohol so that the hemiacetalization is substantially complete, the equilibrium allows conversion substantially to the corresponding monomeric (3-hydrocarbyloxy-3-hydroxypropyl)isocyanurate. Thus, when the aldehyde reactant is the isocyanuric trialdehyde the product is substantially monomeric tris(3-hydrocarbyloxy-3-hydroxypropyl)isocyanurate. However when the amount of the alcohol is less than sufficient to give substantially complete hemiacetalization of the aldehyde groups of the aldehyde, the hemiacetal comprises a mixture of (3-hydrocarbyloxy-3-hydroxypropyl)isocyanurates, (3-hydrocarbyloxy-3-hydroxypropyl) (3-oxopropyl)-isocyanurates, and (3-oxopropyl)isocyanurates. Preferably at least about 0.5 mole of monohydric alcohol is added per mole of combined acrolein for hemiacetal formation.

In the polymer composition of the invention comprising a polyfunctional hemiacetal-reactive compound and a hemiacetal, the preferred hemiacetal-reactive compounds may contain reactive groups such a primary or secondary amines, primary and secondary amides, alcohols, thiols, phenols, anilines and oxiranes.

Crosslinking or polymerization reactions are readily achieved with the hemiacetals of monohydric alcohols by reacting them with polyols under strong acid conditions to produce polyacetals. From what has been said heretofore, the hemiacetals of the monoaldehyde will be effective but the di- and tri-aldehydes are preferred since they can provide more efficient crosslinking. The temperature of the reaction is not critical and is selected, preferably in the range of about 80 to about 150°C to provide the appropriate degree of reaction in a convenient time. Suitable acid catalysts are generally strong acids of pKa less than about 3 such as the mineral acids, oxalic acid, formic acid and acids which are readily soluble in organic systems such as the alkyl-, fluoroalkyl- and aryl-sulfonic acids and the fluorophosphonic acids. Examples of such acids include methanesulfonic acid, trifluoromethanesulfonic acid, benzenesulfonic acid, p-toluenesulfonic acid and hexafluorophosphoric acid.

The reaction is carried out in a solvent inert to the reactants. When the polyols are of molecular weight of about 10,000 or less, high solids coating systems can be obtained containing at least about 50 percent solids and more preferably at least about 70 solids, the solvent being the monohydric alcohol used in hemiacetalization.

Suitable polyols for the acetalization reaction with the monomeric hemiacetals of the (3-oxopropyl)-isocyanurates include oligomeric and polymeric hydroxy compounds containing a plurality of hydroxy

groups per molecule, such as hydroxy-containing polyesters and alkyds, polyethers such as polyethylene glycols, polypropylene glycols and polytetramethylene glycols, polyvinyl alcohol and copolymers of vinyl alcohol, polymers of allyl alcohol, polymers of hydroxyacrylates such as 2-hydroxyethyl acrylate and 2-hydroxyethyl methacrylate, starches, starch derivatives, celluloses and cellulose derivatives. Also suitable are the simple polyols such as 1,4-butanediol, 1,6-hexanediol, 1,4-di(hydroxymethyl)cyclohexane, cyclohexanediols, trimethylolethane, trimethylolpropane, glycerol, pentaerythritol, sorbitol, glucose and sucrose. Diols with hydroxy groups in 1,2- or 1,3-placement, such as ethylene glycol and 1,3-propanediol, which form cyclic acetals may be used as monofunctional reactants for control of the molecular weight of the polyacetals.

Suitable polythiols for reaction with the hemiacetals of the present invention include low molecular weight di- and tri-thiols such as 1,2-ethanedithiol, 1,4-butanedithiol, and 1,6-hexanedithiol, and high molecular weight polythiols such as poly(ethylene thiol), poly(p-styrenethiol) and poly(4-mercaptomethylstyrene).

Among the polyamines which can be reacted with the hemiacetals of the present invention are low molecular weight hydrocarbyldiamines and polyamines and poly(hydrocarbylpolyamines) such as 1,2-ethanediamine, 1,6-hexanediamine and 1,12-dodecanediamine, diethylene triamine and tetrathylene pentamine, and intermediate and high molecular weight poly(hydrocarbylpolyamines), poly(alkylenimines) and vinylamine polymers.

Polyamides suitable for the reaction with the hemiacetals of the present invention include urea, guanidine, the polyamides of polycarboxylic acid such as adipamide and dimer acid diamide, and high molecular weight polyamides such as the nylons, polypeptides and polyacrylamide.

Polyoxiranes which can be used for reaction with the hemiacetals of the present invention include those set forth in the Encyclopaedia of Polymer Science and Technology, Wiley 1967, Vol. 6, pp. 212—219 and include the diglycidyl ether of bisphenol-A and oligomers thereof, epoxidized novolacs, aliphatic polyglycidyl ethers, and peracetic epoxidized products such as vinyl cyclohexene dioxide and epoxidized polybutadiene.

Where m is the functionality of the hemiacetal, based on the effective number of hemiacetal and aldehyde groups per isocyanurate ring, the assumption being made that the aldehyde groups behave as if they are not associated or polymerized, and n is the functionality of the hemiacetal-reactive compound, it being a necessary condition that both m and n be about 2 or greater if chain building reaction is to occur, the mole ratio of hemiacetal to polyfunctional compound for effective polymerization or cure is selected in the range of about 1:m/2 to about n/2:1. The hemiacetal groups can be monofunctional or difunctional depending upon the type and placement of the hemiacetal-reactive groups with which the hemiacetal is reacted. Thus the hemiacetal group will be monofunctional when it is reacted with a primary amine, or with a polyol with hydroxy groups in 1,2- or 1,3 placement to one another to form a cyclic acetal, or when the reaction is limited to a condensation reaction involving the hydroxyl group of the hemiacetal. On the other hand, the hemiacetal group will be difunctional when it reacts with a hemiacetal-reactive compound by condensation reactions in which water and monohydric alcohol are split out as in acetalization of a polyol or mercaptalization of a thiol under strong acid catalysis.

The hemiacetals in which substantially complete hemiacetalization has been achieved with monohydric alcohols, provide a convenient stable source of essentially monomeric (3-oxopropyl) isocyanurates for reactions in aqueous media, since they are readily hydrolyzed when they are added to aqueous media containing a catalytic amount of acid.

The polymerizable compositions comprising polyfunctional compounds and the hemiacetals of the present invention can be used in a multiplicity of applications. They can be used as adhesives and laminating agents, bonding resins, surface coating systems and molding resins. They can be modified by the addition of pigments, plasticizers, colorants, dyes, pigment dispersing agents, flow control agents or stabilizers.

The following Examples are set forth in illustration of the invention. Unless otherwise indicated, all parts and percentages are by weight.

Example 1

(a) *Preparation of Isocyanuric Trialdehyde*

To a pressure kettle (rated for 3.45 MPa (500 psi)), 526 parts by weight of freshly distilled acrolein containing 100 parts per million 2,6-di-t-butyl-4-methylphenol, 200 parts per million hydroquinone and 100 parts per million formic acid is charged. To the stirred acrolein is gradually added 646 parts by weight of 99.2% isocyanuric acid, to form a slurry. Another 500 parts by weight of acrolein is added to the stirred slurry, followed by 26 parts by weight of acrolein containing 2.4 parts by weight of triethylamine and 1.1 parts by weight of 90% formic acid dissolved therein. The isocyanuric acid contains 0.2 weight percent ammelide- and ammeline-sulfamic acids.

The kettle is sealed, and the stirred reactants are heated gradually to 90°C over a 25-minute period. An exotherm occurs at about 90°C; the initial reaction pressure is 170—210 kPa. Reaction temperature is controlled between 89—91°C by steady cooling through the cooling coils of the kettle. Controlled reaction is maintained at 89—91°C. for a total of 45 minutes. However, after 35—40 minutes of reaction, a noticeable decrease in the exothermic rate is observed via the decreased amount of cooling required. The final reaction pressure is about 35 kPa.

After 45 minutes at 90°C, the reaction is terminated by cooling rapidly. Simultaneously 1500 parts of methyl ethyl ketone containing 5 parts of 90% formic acid is pumped into the stirred contents and stirring is continued with cooling until the temperature is ~20°C.

The reaction solution is drained from the kettle. It is filtered once through a Celite bed on a Buchner funnel; and twice through filter paper on a Buchner funnel. To the clear yellow filtrate then is added 15.0 parts of oxalic acid and the solution is stripped in a rotating evaporator at a water bath temperature of 60°C and an absolute pressure of 120 to 200 mg Hg. When the solvent has been removed by distillation, 800 parts of fresh methyl ethyl ketone is added to dissolve the product. The same stripping procedure is followed. At the end of the second stripping operation, the bath temperature is raised rapidly to 100°C and maintained at that temperature for about 5 minutes. The residual product, a turbid viscous liquid of 3.500 Pa s viscosity at 80°C, then is mixed with 1500 parts of water and the solution is stripped at 105°C/180—18 mm/30 min to produce the product. This washing procedure is repeated twice more. The final product is poured into an aluminum tray. When allowed to cool to room temperature, it rapidly hardens to a glassy solid which crystallizes or becomes opaque after 1—48 hours. 1506 parts by weight of product are obtained corresponding to a yield of 100%. The opaque white, glassy solid can be ground up to a white powder. The ratio of aldehyde carbonyl infrared absorbance at 1740 $cm^{-1}$ to isocyanurate absorbance at 780 $cm^{-1}$ is 1.8. The aldehyde content determined by the quantitative hydroxylamine method corresponds to an equivalent weight of 101.6. The residual acrolein monomer, determined by gas chromatography is less than 100 parts per million of product.

When the reaction product is subjected to thin layer chromatography by the general procedure described in Egon Stahl's "Thin Layer Chromatography", Springer-Verlay, Heidelberg-New York, 2nd Ed. 1969 (English Translation) on EM Silica Gel-60-F-254 with ethyl acetate elution (2×) and visualizations by means of UV light (254 nm) for double bonds; dichlorofluoroscein; AHTT aldehyde detection agent; and a solution of chlorine in o-toluidine for NH and $NH_2$ detection, four aldehyde components, 1 major corresponding to tris (3-oxopropyl)isocyanurate and 3 lesser, are separated. Two of the lesser ones may be polymeric. A trace of a fifth component, possibly acrolein oligomer, is also observed.

The method of Example 1 is repeated several times and the reaction products are subjected to analysis by a gel permeation chromatography technique. This method employs two Varian Micropak® TSK columns (sizes 1000H and 2000H in sequence, each 50 cm in length) which can separate materials by size from 100 to about 10,000 molecular weight. Coupled with a UV spectrophotometer detector set at 220 nm wavelength, a tetrahydrofuran solution of the products is separated into 13 components. These consist of 3 groups: polymeric species, isocyanuric acid — acrolein adducts, and acrolein oligomers. Peak height ratios are used to determine relative percentages of the components. The following range is obtained:

| | |
|---|---|
| monomeric isocyanuric-acrolein adducts | 39—62% |
| polymer component | 39—58% |
| acrolein oligomers | 0.2—3.9% |

The range of tris-, bis- and mono-(3-oxopropyl) isocyanurates in the monomeric component are determined to be:

| | |
|---|---|
| tris-(3-oxopropyl)isocyanurate | 39—71% |
| bis-(3-oxopropyl)isocyanurate | 21—29% |
| mono-(3-oxopropyl)isocyanurate | 4—14% |

The precision of the analysis for the monomeric and polymeric components is ±4%.

$^{13}C$ NMR analysis of the reaction product displays the following peaks:

| Peak (ppm) | Assignment |
|---|---|
| 200 | saturated aldehyde carbonyl |
| 148 | isocyanuric acid carbonyl |
| 40.4 | α-methylene of saturated aldehyde |
| 35.8 | methylene attached to ring nitrogen |

Quantitatively, $^{13}C$ NMR has measured ranges of 2.0 to 2.7 aldehydes per isocyanuric acid ring.

(b) *Preparation of Hemiacetal Solutions*

The adduct of acrolein and isocyanuric acid prepared as set forth in (a) above (143 parts by weight) is heated at 50—60°C with 45 parts by weight of methanol until a clear homogeneous liquid forms. The liquid is dried under vacuum at room tmeperature over phosphorous pentoxide for 2 hours. Infra-red analysis of the liquid shows a strong hydroxyl band and a weak aldehyde carbonyl band.

Similarly, solutions of the hemiacetal derivatives of the acrolein-isocyanuric acid adduct are prepared by reaction of the aldehyde with isopropyl alcohol, n-butyl alcohol, 2-ethylhexanol-1, and benzyl alcohol. The infra-red absorption band for aldehyde carbonyl in these products also is significantly less intense than the aldehyde band in the initial acrolein-isocyanuric adduct. The aldehyde carbonyl band is less intense in the spectra of hemiacetal products of the higher boiling alcohols in comparison with the hemiacetal products of the lower boiling alcohols, suggesting that the equilibrium is less readily shifted back to the aldehyde because of the lower volatility of such alcohols.

Little reaction is observed between t-butyl alcohol and the tris(3-oxopropyl)isocyanurate material. The latter did not go into solution in t-butyl alcohol even after prolonged heating at 80°C.

Example 2

Preparation of a Tris(3-hydroxy-3-methoxypropyl)isocyanurate Composition

The procedure of Example 1 (a) is repeated except that methyl alcohol is substituted for methyl ethyl ketone, and the filtered solution is vacuum dried at 25°C over phosphorous pentoxide to constant weight. An infra-red spectrum showing a strong hydroxyl band and the almost complete disappearance of the aldehyde carbonyl band at 1740 $cm^{-1}$ confirms the conversion of the polyaldehyde substantially to tris(3-hydroxy-3-methoxypropyl)isocyanurate.

**Claims**

1. A process for the preparation of a hemiacetal which comprises:

a. slurrying isocyanuric acid in a solvent medium having a pH in the range of about 5 to about 7, the said medium containing at least about 1 mole of acrolein per mole of isocyanuric acid;

b. allowing the acrolein to add to the isocyanuric acid at a temperature in the range of about 50 to about 120°C to form a substantially clear solution; and

c. allowing the adduct to react with a monohydric primary or secondary alcohol at a temperature in the range of about 50 to about 80°C at a pH in the range of about 5 to about 7.

2. The process of Claim 1 wherein the amount of acrolein, at step (a) is in the range of about 1 to about 6 moles per mole of isocyanuric acid, wherein excess acrolein is removed at the end of step (b) and wherein the amount of monohydric alcohol reacted at step (c) is in the range of about 0.5 to about 1.0 mole per mole of combined acrolein.

3. The process of Claim 1 wherein the reaction at step (b) is carried out at a temperature in the range of about 80 to about 100°C and wherein about one mole of monohydric alcohol per mole of combined acrolein is reacted at step (c).

4. The process of Claim 2 wherein the pH in the range of about 5 to about 7 is obtained by addition of from about 0.1 to about 1.0 mole of an amine salt per 100 moles of isocyanuric acid.

5. The process of Claim 4 wherein the amine salt is triethylamine formate.

6. The process of Claim 1 wherein the monohydric alcohol is a $C_1$ to $C_8$ monohydric alcohol.

7. The process of Claim 2 wherein the monohydric alcohol is methyl alcohol.

8. A hemiacetal of a monohydric primary or secondary alcohol and the acrolein-isocyanuric acid adduct obtainable by the process of claim 1 wherein the adduct comprises from about 1 to about 3 moles of acrolein per mole of isocyanuric acid.

9. The hemiacetal of Claim 8 wherein the monohydric alcohol is a $C_1$ to $C_8$ alcohol.

10. The hemiacetal of Claim 8 wherein the monohydric alcohol is methyl alcohol.

11. A hemiacetal composition comprising a (3-hydroxy-3-hydrocarbyloxypropyl)isocyanurate.

12. A hemiacetal composition comprising tris (3-hydroxy-3-hydrocarbyloxypropyl)isocyanurate.

13. The hemiacetal of Claim 11 or 12 wherein the hydrocarbyl group contains 1 to 8 carbon atoms.

14. The hemiacetal of Claim 11 or 12 wherein the hydrocarbyl group is methyl.

15. A composition comprising a polyfunctional hemiacetal-reactive compound and a hemiacetal of a monohydric primary or secondary alcohol and the acrolein-isocyanuric acid adduct obtainable by the process of claim 1.

16. The composition of Claim 15 wherein the adduct comprises from about 1 to about 3 moles of acrolein added per mole of isocyanuric acid and is hemiacetalized with from about 0.5 to about 1 mole of monohydric alcohol per mole of combined acrolein.

17. The composition of Claim 15 wherein the monohydric alcohol is a $C_1$ to $C_8$ alcohol.

18. The composition of Claim 15 wherein the monohydric alcohol is methyl alcohol.

19. The composition of Claim 15, 16, 17 or 18 wherein the mole ratio of hemiacetal to polyfunctional hemiacetal-reactive compound is in the range of about 1:m/2 to about n/2:1 where m is the functionality of the hemiacetal and n is the functionality of the polyfunctional hemiacetal-reactive compound and m and n are at least about 2.

20. The composition of Claim 19 wherein the functional groups of the hemiacetal-reactive compound are selected from the group consisting of primary and secondary amine, primary and secondary amide, alcohol, thiol and oxirane.

21. The composition of Claim 19 wherein the hemiacetal-reactive compound is a polyhydroxy compound.

22. The reaction product of the composition of Claim 19, 20 or 21.

23. An article comprising the reaction product of the composition of Claim 19, 20 or 21.

**Patentansprüche**

1. Verfahren zur Herstellung eines Halbacetals, dadurch gekennzeichnet, daß man

a) Isocyanursäure in einem Lösungsmittelmedium mit einem pH-Wert im Bereich von etwa 5 bis etwa 7, welches Medium mindestens etwa 1 Mol Acrolein pro Mol Isocyanursäure enthält, aufschlämmt;

b) die Additionsreaktion des Acroleins an die Isocyanursäure bei einer Temperatur im Bereich von etwa 50 bis etwa 120°C unter Bildung einer im wesentlichen klaren Lösung ablaufen läßt; und

c) das Addukt bei einer Temperatur im Bereich von etwa 50 bis etwa 80°C bei einem pH-Wert im Bereich von etwa 5 bis etwa 7 mit einem einwertigen primären oder sekundären Alkohol reagieren läßt.

2. Verfahren nach Anspruch 1, dadurch gekennzeichnet, daß die in der Stufe (a) verwendete Acroleinmenge im Bereich von etwa 1 bis etwa 6 Mol pro Mol Isocyanursäure liegt, wobei das überschüssige Acrolein am Ende der Stufe (b) entfernt wird, und die Menge des in der Stufe (c) umgesetzten einwertigen Alkohols im Bereich von etwa 0,5 bis etwa 1,0 Mol pro Mol des gebunden Acroleins liegt.

3. Verfahren nach Anspruch 1, dadurch gekennzeichnet, daß die Reaktion in der Stufe (b) bei einer Temperatur im Bereich von etwa 80 bis etwa 100°C durchgeführt wird und etwa 1 Mol des einwertigen Alkohols pro Mol des gebundenen Acroleins in der Stufe (c) umgesetzt wird.

4. Verfahren nach Anspruch 2, dadurch gekennzeichnet, daß der pH-Wert im Bereich von etwa 5 bis etwa 7 durch Zugabe von etwa 0,1 bis etwa 1,0 Mol eines Aminsalzes pro 100 Mol Isocyanursäure eingestellt wird.

5. Verfahren nach Anspruch 4, dadurch gekennzeichnet, daß man als Aminsalz Triethylaminformiat einsetzt.

6. Verfahren nach Anspruch 1, dadurch gekennzeichnet, daß man als einwertigen Alkohol einen einwertigen $C_1$—$C_8$-Alkohol verwendet.

7. Verfahren nach Anspruch 2, dadurch gekennzeichnet, daß man als einwertigen Alkohol Methylalkohol einsetzt.

8. Halbacetal aus einem einwertigen primären oder sekundären Alkohol und dem Acrolein-Isocyanursäure-Addukt erhältlich nach dem Verfahren gemäß Anspruch 1, wobei das Addukt etwa 1 bis etwa 3 Mol Acrolein pro Mol Isocyanursäure umfaßt.

9. Halbacetal nach Anspruch 8, dadurch gekennzeichnet, daß der einwertige Alkohol ein $C_1$—$C_8$-Alkohol ist.

10. Halbacetal nach Anspruch 8, dadurch gekennzeichnet, daß der einwertige Alkohol Methylalkohol ist.

11. Halbacetalprodukt enthaltend ein (3-Hydroxy-3-hydrocarbyloxypropyl)-isocyanurat.

12. Halbacetalprodukt enthaltend Tris(3-hydroxy-3-hydrocarbyloxypropyl)-isocyanurat.

13. Halbacetal nach den Ansprüchen 11 oder 12, dadurch gekennzeichnet, daß die Hydrocarbylgruppe (Kohlenwasserstoffgruppe) 1 bis 8 Kohlenstoffatome enthält.

14. Halbacetal nach den Ansprüchen 11 oder 12, dadurch gekennzeichnet, daß die Hydrocarbylgruppe eine Methylgruppe ist.

15. Zubereitung enthaltend eine polyfunktionelle, mit einem Halbacetal reagierende Verbindung und ein Halbacetal aus einem einwertigen primären oder sekundären Alkohol und dem Acrolein-Isocyanursäure-Addukt erhältlich nach dem Verfahren gemäß Anspruch 1.

16. Zubereitung nach Anspruch 15, dadurch gekennzeichnet, daß das Addukt etwa 1 bis etwa 3 Mol des an Isocyanursäure gebundenen Acroleins pro Mol Isocyanursäure umfaßt und mit etwa 0,5 bis etwa 1 Mol des einwertigen Alkohols pro Mol des gebundenen Acroleins in das Halbacetal umgewandelt worden ist.

17. Zubereitung nach Anspruch 15, dadurch gekennzeichnet, daß der einwertige Alkohol ein $C_1$—$C_8$-Alkohol ist.

18. Zubereitung nach Anspruch 15, dadurch gekennzeichnet, daß der einwertige Alkohol Methylalkohol ist.

19. Zubereitung nach den Ansprüchen 15, 16, 17 oder 18, dadurch gekennzeichnet, daß das Molverhältnis von Halbacetal zu der polyfunktionellen, mit einem Halbacetal reagierenden Verbindung im Bereich von etwa 1:m/2 bis etwa n/2:1 liegt, worin m für die Funktionalität des Halbacetals und n für die Funktionalität der polyfunktionellen, mit einem Halbacetal reagierenden Verbindung stehen und m und n einen Wert von mindestens etwa 2 besitzen.

20. Zubereitung nach Anspruch 19, dadurch gekennzeichnet, daß die funktionellen Gruppen der mit dem Halbacetal reagierenden Verbindung aus der Gruppe ausgewählt sind, die primäre und sekundäre Amingruppen, primäre und sekundäre Amidgruppen, Alkoholgruppen, Thiolgruppen und Oxirangruppen umfaßt.

21. Zubereitung nach Anspruch 19, dadurch gekennzeichnet, daß die mit dem Halbacetal reagierende Verbindung eine Polyhydroxyverbindung ist.

22. Reaktionsprodukt der Zubereitung gemäß den Ansprüchen 19, 20 oder 21.

23. Artikel enthaltend das Reaktionsprodukt der Zubereitungen gemäß den Ansprüchen 19, 20 oder 21.

**Revendications**

1. Procédé pour la préparation d'un hémiacétal, qui consiste à:

a - délayer de l'acide isocyanurique dans un milieu solvant présentant un pH compris entre environ 5 et environ 7, le milieu contenant au moins environ 1 mole d'acroléine par mole d'acide isocyanurique;

b - laisser l'acroléine se fixer par addition sur l'acide isocyanurique à une température comprise entre environ 50 et environ 120°C pour former une solution pratiquement transparente; et

c - laisser le produit d'addition réagir avec un monoalcool primaire ou secondaire à une température comprise entre environ 50 et environ 80°C, à un pH compris entre environ 5 et environ 7.

2. Procédé selon la revendication 1, caractérisé en ce que la quantité d'acroléine dans l'étape (a) est comprise entre environ 1 et environ 6 moles par mole d'acide isocyanurique, en ce qu'on élimine l'acroléine en excès à la fin de l'étape (b) et en ce que la quantité de monoalcool qui réagit dans l'étape (c) est comprise entre environ 0,5 et environ 1,0 mole par mole d'acroléine combinée.

3. Procédé selon la revendication 1, caractérisé en ce qu'on met en oeuvre la réaction, dans l'étape (b) à une température comprise entre environ 80 et environ 100°C, et en ce qu'on fait réagir environ une mole de monoalcool par mole d'acroléine combinée dans l'étape (c).

4. Procédé selon la revendication 2, caractérisé en ce qu'on obtient le pH compris entre environ 5 et environ 7 par addition d'environ 0,1 à environ 1,0 mole d'un sel d'amine pour 100 moles d'acide isocyanurate.

5. Procédé selon la revendication 4, caractérisé en ce que le sel d'amine est le formiate de triéthylamine.

6. Procédé selon la revendication 1, caractérisé en ce que le monoalcool est un monoalcool en $C_1$ à $C_8$.

7. Procédé selon la revendication 2, caractérisé en ce que le monoalcool est l'alcool méthylique.

8. Hémiacétal d'un monoalcool primaire ou secondaire et du produit d'addition acroléine acide isocyanurique que l'on peut obtenir selon la procédé de la revendication 1, caractérisé en ce que le produit d'addition comprend d'environ 1 à environ 3 moles d'acroléine par mole d'acide isocyanurique.

9. Hémiacétal selon la revendication 8, caractérisé en ce que le monoalcool est un alcool en $C_1$ à $C_8$.

10. Hémiacétal selon la revendication 8, caractérisé en ce que le monoalcool est l'alcool méthylique.

11. Composition d'hémiacétal comprenant un isocyanurate de (3-hydroxy-3-hydrocarbyloxypropyle).

12. Composition d'hémiacétal comprenant un isocyanurate de tris-(3-hydroxy-3-hydrocarbyloxypropyle).

13. Hémiacétal selon la revendication 11 ou 12, caractérisé en ce que le groupe hydrocarbyle contient de 1 à 8 atomes de carbone.

14. Hémiacétal selon la revendication 11 ou 12, caractérisé en ce que le groupe hydrocarbyle est le groupe méthyle.

15. Composition comprenant un composé polyfonctionnel réagissant avec les hémiacétals et un hémiacétal d'un monoalcool primaire ou secondaire et du produit d'addition acroléine-acide isocyanurique que l'on peut obtenir selon le procédé de la revendication 1.

16. Composition selon la revendicaton 15, caractérisée en ce que le produit d'addition comprend d'environ 1 à environ 3 moles d'acroléine fixée par addition par mole d'acide isocyanurique et en ce qu'il est hémiacétalisé avec d'environ 0,5 à environ 1 mole de monoalcool, par mole d'acroléine combinée.

17. Composition selon la revendication 15, caractérisée en ce que le monoalcool est un alcool en $C_1$—$C_8$.

18. Composition selon la revendication 15, carctérisée en ce que le monoalcool est l'alcool méthylique.

19. Composition selon la revendication 15, 16, 17 ou 18, caractérisée en ce que le rapport molaire de l'hémiacétal au composé polyfonctionnel réagissant avec les hémiacétals est compris entre environ 1:m/2 et environ n/2:1, sachant que m représente le nombre de groupes fonctionnels de l'hémiacétal et n le nombre de groupes fonctionnels du composé polyfonctionnel réagissant avec les hémiacétals, m et n étant au moins égaux à 2.

20. Composition selon la revendication 19, caractérisée en ce que les groupes fonctionnels du composé réagissant avec les hémiacétals sont choisis dans le groupe constitué par des amines primaires et secondaires, des amides primaires et secondaires, des alcools, des thiols et des oxirannes.

21. Composition selon la revendication 19, caractérisée en ce que le composé réagissant avec les hémiacétals est un composé polyhydroxylé.

22. Produit de réaction de la composition selon la revendication 19, 20 ou 21.

23. Article comprenant le produit de réaction de la composition selon la revendication 19, 20 ou 21.